(19) Europäisches Patentamt
European Patent Office
Office européen des brevets

(11) Veröffentlichungsnummer: **0 212 100**
**B1**

(12) **EUROPÄISCHE PATENTSCHRIFT**

(45) Veröffentlichungstag der Patentschrift: **11.04.90**

(51) Int. Cl.⁵: **A 61 M 5/00**

(21) Anmeldenummer: **86107567.9**

(22) Anmeldetag: **04.06.86**

(54) **Vorrichtung mit zwei miteinander zu vermischenden Lösungen.**

(30) Priorität: **25.06.85 DE 3522645**

(43) Veröffentlichungstag der Anmeldung:
**04.03.87 Patentblatt 87/10**

(45) Bekanntmachung des Hinweises auf die Patenterteilung:
**11.04.90 Patentblatt 90/15**

(84) Benannte Vertragsstaaten:
**AT BE CH FR GB IT LI NL SE**

(56) Entgegenhaltungen:
**AT-B- 373 150**
**DE-A-2 951 106**
**DE-A-3 238 649**
**GB-A- 911 277**
**US-A-3 459 182**
**US-A-3 788 369**
**US-A-4 233 973**
**US-A-4 458 733**

(73) Patentinhaber: **Laboratorien Hausmann AG**
**Rechenstrasse 37**
**CH-9001 St. Gallen (CH)**

(72) Erfinder: **Geisser, Peter, Dr. rer. nat.**
**Marweesstrasse 8**
**CH-9014 St. Gallen (CH)**
Erfinder: **Kaiser, Hans Peter, Dr. sc. nat.**
**Steinhaldenstrasse 66**
**CH-8002 Zürich (CH)**
Erfinder: **Berger, Franz**
**Lerchenstrasse 31**
**CH-9202 Gossau (CH)**

(74) Vertreter: **Türk, Gille, Hrabal**
**Brucknerstrasse 20**
**D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 212 100 B1**

**Beschreibung**

Die Erfindung betrifft eine Vorrichtung mit zwei unmittelbar vor dem Verabreichen steril miteinander zu vermischenden aufbewahrten Flüssigkeiten mit den Merkmalen des Oberbegriffes des Patentanspruchs 1.

Häufig ist es notwendig, parenteral zu verabreichende Lösungen, beispielsweise Infusionslösungen, erst unmittelbar vor der Verabreichung miteinander zu vermischen, da einerseits manche Mischungen nicht über längere Zeit lagerbeständig sind und andererseits häufig der Arzt erst entscheidet, welche Mischungen im einzelnen Fall verabreicht werden sollen.

Bei einer bekannten Vorrichtung dieser Art (AT-B-373 150) ist es erforderlich, um die in den Behältern befindlichen Flüssigkeiten zu vermischen, die Höhenlage der einen Flasche in Bezug auf die andere zu verändern. Bei kollabierbaren Kunststoffbehältern ist es bei der bekannten Vorrichtung notwendig, die Flüssigkeit des jeweiligen zu entleerenden Behälters durch Zusammendrükken des Behälters aus diesem in den anderen zu überführen. Um ein Vermischen der Flüssigkeiten zu erreichen, ist somit eine zusätzliche manuelle oder aber mechanische Beeinflussung der Behälter, sei es in ihrer Lage zueinander oder aber in ihrer Form vonnöten. Desweiteren bedarf es des öfteren zu einer intensiven und gleichförmigen Vermischung der Flüssigkeiten einer Wiederholung dieser Tätigkeit, d.h., die Lösung wird einmal von der einen Flasche in die andere und umgekehrt gedrückt. Hinzukommt daß zur Halterung bzw. Handhabung der beiden die Flüssigkeiten aufbewahrenden Behälter diese getrennt voneinander gehandhabt werden müssen d.h., eine Halterung vonnöten ist, die beide Behälter trägt, so daß die Handhabung in einem etwaigen Notfall sich als zu umständlich und zeitraubend für eine Person darstellt.

Weiterhin hat man in den Fällen, in denen zwei Infusionslösungen zu verabreichen waren, die beiden Lösungen beispielsweise getrennt verabreicht. Ebenso ist es bekannt, die beiden Lösungen in einem Doppelbeutel aus Kunststoff unterzubringen, der zwei oder mehr über eine aufreißbare Verbindung zunächst voneinander abgeschottete Behälterteile zur Aufnahme der einzelnen Lösungen aufweist (DE-32 38 649 A1). Vor der Verabreichung wird die Verbindung zwischen den einzelnen Behälterteilen aufgerissen, um die in den Behälterteilen befindlichen Flüssigkeiten miteinander vermischen zu können. Zu diesem Zweck muß der Beutel beispielsweise von Hand gewalkt werden, um eine auch nur einigermaßen gleichförmige Durchmischung der Lösung zu erzielen. Eine vollständige Durchmischung ist kaum zu erreichen.

Das getrennte Verabreichen von zwei Lösungen erfordert ebenso wie das Vermischen von in einem Doppelbeutel untergebrachten Flüssigkeiten mechanische Maßnahmen, die aufwendig sind, jedoch nicht zu einer völlig gleichförmigen Mischung führen. Hingegen besteht beispielsweise im Falle einer Infusion die Gefahr, daß das Infusionsbesteck durch die mechanische Vermischbewegung verrutschen kann. Bei Verwendung von Glasrohrverbindungen besteht zudem die Gefahr, daß Glasbruch in die zu verabreichenden Lösungen gelangt.

Manche parenteral zu verabreichenden Lösungen können nicht in Kunststoffbehältern und dementsprechend nicht in flexiblen Kunststoffbeuteln aufbewahrt werden, da eine Wechselwirkung zwischen dem Kunststoff des Behältnisses oder dem vom Kunststoff durchgelassenen Luftsauerstoff und den Wirkbestandteilen der Lösung eintreten kann. Dies ist beispielsweise bei Aminosäurelösungen der Fall.

Aufgabe der Erfindung ist es, eine Vorrichtung zu schaffen, in der zwei Lösungen völlig getrennt und ohne die Gefahr einer unerwünschten vorzeitigen Vermischung aufbewahrt, jedoch vor dem Verabreichen ohne die Notwendigkeit zusätzlicher mechanischer Beeinflussung intensiv und gleichförmig miteinander vermischt werden können.

Diese Aufgabe wird erfindungsgemäß bei einer Vorrichtung der eingangs genannten Gattung mit den Merkmalen des kennzeichnenden Teiles des Patentanspruches 1 gelöst. Weitere vorteilhafte Ausgestaltungen der Erfindung sind Gegenstand der Unteransprüche.

Dadurch, daß gemäß der Erfindung der erste Behälter der erfindungsgemäßen Vorrichtung ein flexibler Beutel aus Kunststoff ist, daß der flexible Behälter mit zwei zum Aufstecken auf den starren Behälter bestimmten Laschen, die Öffnungen enthalten, welche den starren Behälter aufnehmen können, und ferner mit einer zum Aufhängen der Vorrichtung geeigneten Schlaufe versehen ist und der zweite Behälter ein separater vakuumdicht verschließbarer starrer Behälter ist, der ein derartiges Fassungsvermögen bzw. Volumen aufweist, daß er das Gesamtvolumen beider Flüssigkeiten zum Vermischen derselben aufnehmen kann, ist gewährleistet, daß dadurch daß der starre Behälter nach dem Einfüllen der einen Flüssigkeit oder Lösung evakuiert und vakuumdicht verschlossen wurde, die im flexiblen Beutel befindliche Flüssigkeit von dem im starren Behälter herrschenden Unterdruck in den starren Behälter eingesaugt wird, sobald mit dem speziellen Infusionsbesteck eine Verbindung zwischen dem flexiblen Beutel und dem starren Behälter hergestellt worden ist. Der im starren Behälter herrschende Unterdruck sorgt nicht nur für ein vollständiges Einsaugen der Flüssigkeit aus dem flexiblen Behälter, sondern auch für ein Vermischen der beiden Flüssigkeiten oder Lösungen im starren Behälter, der dann wie eine übliche Infusionsflasche umgekehrt aufgehängt werden kann, um die in ihm gebildete Infusionslösung in üblicher Weise einem Patienten zu verabreichen.

Da sowohl der flexible Beutel als auch der starre Behälter steril verschlossen sind, ist ein steriles Vermischen der beiden Flüssigkeiten unmittelbar vor dem Verabreichen möglich. Dementsprechend kann man in einfacher Weise zwei beispielsweise parenteral zu verabreichende Flüssigkeiten ohne umständliche mechanische Maßnahme wie Quetschen oder Walken von Kunststoffbeuteln, Zerbrechen oder Aufreißen von Trennwänden, Anheben und Schütteln von Behältern und dergleichen steril miteinander vermischen. Dabei ergibt sich auch die Möglichkeit, erst unmittelbar vor dem Vermischen zu entscheiden, welche Lösungen bzw. Lösungsbestandteile oder Lösungsmengen miteinander vermischt werden sollen, weil der flexible Beutel und der starre Behälter der erfindungsgemäßen Vorrichtung erst dann endgültig miteinander verbunden werden, wenn das Infusionsbesteck in deren Stopfen-Verschlüsse eingesteckt worden ist.

Der starre Behälter wird im allgemeinen etwas mehr als zur Hälfte mit einer der Lösungskomponenten wie einer Aminosäurelösung unter sterilen Bedingungen gefüllt, evakuiert und verschlossen, weil im allgemeinen eine kleinere Menge Glucoselösung mit einer größeren Menge Aminosäurelösung zu vermischen ist. Dabei kann das Volumen des starren Behälters etwas größer als das Volumen des Flüssigkeitsgemisches sein, um eine problemlose und gleichförmige Durchmischung der Flüssigkeitskomponenten zu gewährleisten, nachdem die Verbindung zwischen den die einzelnen Flüssigkeitskomponenten aufnehmenden Behältnissen hergestellt worden ist.

Desweiteren ist so sichergestellt, daß der flexible Kunststoffbeutel derart auf den starren Behälter aufsetzbar ist, daß beide zusammen eine transportable Einheit bilden, ohne daß ihre Innenräume in Verbindung miteinander kommen könnten, bevor eine solche Verbindung nicht mit Hilfe des Infusionsbesteckes hergestellt wird.

Dabei ist der flexible Beutel vorzugsweise so ausgebildet, daß er ähnlich wie ein Rucksack sozusagen "Huckepack" auf den starren Behälter wie die Infusionsflasche aufgesteckt werden kann. Desweiteren ist es durch die Ausgestaltung des flexiblen Beutels möglich, die Vorrichtung mit Hilfe der Lasche mit nur einer Hand zu tragen und die Infusionsflasche mit dem aufgesteckten Kunststoffbeutel mit der Auslaßöffnung nach unten hochzuheben und beispielsweise an einen Haken für die Infusionszeit anhängen zu können.

Obwohl die Erfindung in Verbindung mit dem Vermischen von zwei Flüssigkeiten zu einer Lösung beschrieben ist, können auch mehr als zwei Flüssigkeiten miteinander vermischt werden, wobei der vakuumdicht verschließbare starre Behälter ein ausreichendes Volumen zur Aufnahme aller Teilflüssigkeiten aufweisen muß, die in einer entsprechenden Anzahl von flexiblen Beuteln steril aufbewahrt werden.

Durch die Anordnung der zur Aufnahme des starren Behälters bestimmten Laschen an entgegengesetzten Enden des rechteckig ausgebildeten flexiblen Behälters und dadurch daß sich neben der einen Lasche der Stopfen-Verschluß des flexiblen Behälters befindet, ist eine besonders gute Handhabbarkeit, d.h. eine besonders einfache und praktikable Anbringungsmöglichkeit des flexiblen Behälters an dem starren Behälter gewährleistet und desweiteren eine ebenso einfache und gute Handhabbarkeit des Infusionsbesteckes in Verbindung mit den beiden die Flüssigkeiten beinhaltenden Behälter.

Ist der starre Behälter flaschenförmig ausgebildet und enthält die neben dem Stopfen-Verschluß des flexiblen Behälters befindliche Lasche eine zur Aufnahme des Flaschenhalses des starren Behälters bestimmte Öffnung, die enger als die zur Aufnahme des Bauches des starren Behälters bestimmte Öffnung in der anderen Lasche ist, so ist dadurch ein sicheres Aneinanderhalten der beiden Behälter gewährleistet.

Besteht der starre Behälter aus einer Glasflasche, so stellt dies eine besonders günstige und preiswerte Herstellungsmöglichkeit des starren Behälters sicher und bietet zum andern auch die Sicherheit, daß eine Wechselwirkung zwischen Behälter und darin aufzubewahrenden Flüssigkeiten ausgeschlossen ist.

Der flexible Beutel besteht aus thermoplastischen Kunststoff wie beispielsweise Polyvinylchlorid. Die erfindungsgemäße Vorrichtung ist besonders für miteinander zu einer Infusionslösung zu vermischenden Glucoselösungen und Aminosäurelösungen geeignet. Es ist bekannt, aus derartigen Bestandteilen Infusionslösungen herzustellen, wobei auch bekannt ist, daß Glucoselösungen und Aminosäurelösungen vor der Verabreichung voneinander getrennt gelagert werden müssen, um die sogenannte Maillard-Reaktion zwischen den Hydroxylgruppen der Glucose oder eventuell anderer Zucker wie z.B. Fructose einerseits und den Aminogruppen der Aminosäuren zu vermeiden.

Die Aminosäurelösungen bestehen in der Regel aus Gemischen verschiedener für den Organismus notwendiger Aminosäuren gemäß der Art der Behandlung des Patienten. Die Aminosäurelösungen enthalten in der Regel wenigstens die acht sogenannten essentiellen Aminosäuren sowie gegebenenfalls Histidin (z.B. bei Nierenerkrankungen). Bei einer totalen parenteralen Versorgung kann die Aminosäurelösung aber auch die sogenannten halb/essentiellen/oder nichtessentiellen Aminosäuren enthalten. Die Konzentration der Aminosäuren in der Aminosäurelösung liegt im allgemeinen im Bereich von etwa 0,5 bis 30 g pro 100 ml Lösung. Die untere Grenze liegt häufig bei etwa 3 g pro 100 ml und die obere bei etwa 20 g pro 100 ml.

Die Konzentration der Zuckerlösung, insbesondere Glucoselösung, liegt in der Regel im Bereich von etwa 5 bis 90 g pro 100 ml der Zuckerlösung. Die untere Grenze liegt in der Regel bei etwa 20 g pro 100 ml und die obere bei etwa 85 g pro 100 ml, wobei Lösungen mit einem Gehalt in der Größenordnung von etwa 70 bis 80 g pro 100 ml Glucose bevorzugt sind.

Dies ist dem Fachmann grundsätzlich bekannt (vgl. Total Parenteral Nutrition in the Hospital and at Home; K. N. Jeejeebhoy, CRC Press Inc. Boca Raton, Florida, USA (1983)).

Bei Verwendung der erfindungsgemäßen Vorrichtung zum Aufbewahren und Vermischen von Glucoselösungen und Aminosäurelösungen wird die Aminosäurelösung in den vakuumdicht verschließbaren starren Behälter, vorzugsweise die Glasflasche, eingefüllt, da gefunden wurde, daß Aminosäurelösungen nicht in Kunststoffbeuteln aufbewahrt werden können, weil die Gefahr besteht, daß Aminosäurelösungen durch den Kunststoff und/oder eindringenden Luftsauerstoff verändert werden.

Die halbkreisförmige Ausbildung, der als Aufhängeeinrichtung dienenden Schlaufe erweist sich sowohl in der Handhabung als auch in der Haltbarkeit als äußerst praktikabel.

Befindet sich die halbkreisförmige Schlaufe in der Ebene der Lasche mit der größeren Öffnung und über diese Lasche überstehend so wird dadurch zum einen die einfache Handhabbarkeit der Vorrichtung gefördert, da zum Aufhängen der Flasche mit dem Stopfen-Verschluß nach unten der Handgriff, d.h., die halbkreisförmige Schlaufe benutzt werden kann ohne daß die Gefahr des Entgleitens der Infusionsflasche bzw. Vorrichtung besteht.

In der Zeichnung ist ein Ausführungsbeispiel der erfindungsgemäßen Vorrichtung dargestellt, und zwar zeigt

Fig. 1 eine schaubildliche Ansicht einer den starren Behälter der Vorrichtung bildenden Infusionsflasche, die mit einer der miteinander zu vermischenden Flüssigkeiten teilweise gefüllt ist,

Fig. 2 eine Draufsicht auf den für eine zweite Flüssigkeit bestimmten flexiblen Kunststoff-Beutel,

Fig. 3 eine Ansicht des speziellen Infusionsbesteckes der erfindungsgemäßen Vorrichtung,

Fig. 4 eine Ansicht, aus der erkennbar ist, wie der mit der einen Flüssigkeit gefüllte Kunststoff-Beutel auf die mit der anderen Flüssigkeit teilweise gefüllte Infusionsflasche aufgesteckt wird,

Fig. 5 eine schaubildliche Ansicht der Infusionsflasche mit dem huckepack auf dieser angebrachten flexiblen Kunststoff-Beutel, wobei zu erkennen ist, wie das erfindungsgemäße Infusionsbesteck an die Infusionsflasche und den flexiblen Beutel angeschlossen wird, und

Fig. 6 eine schaubildliche Ansicht der erfindungsgemäßen Vorrichtung in der Betriebsstellung, in der die in der Infusionsflasche gemischte Infusionslösung einem Patienten verabreicht wird.

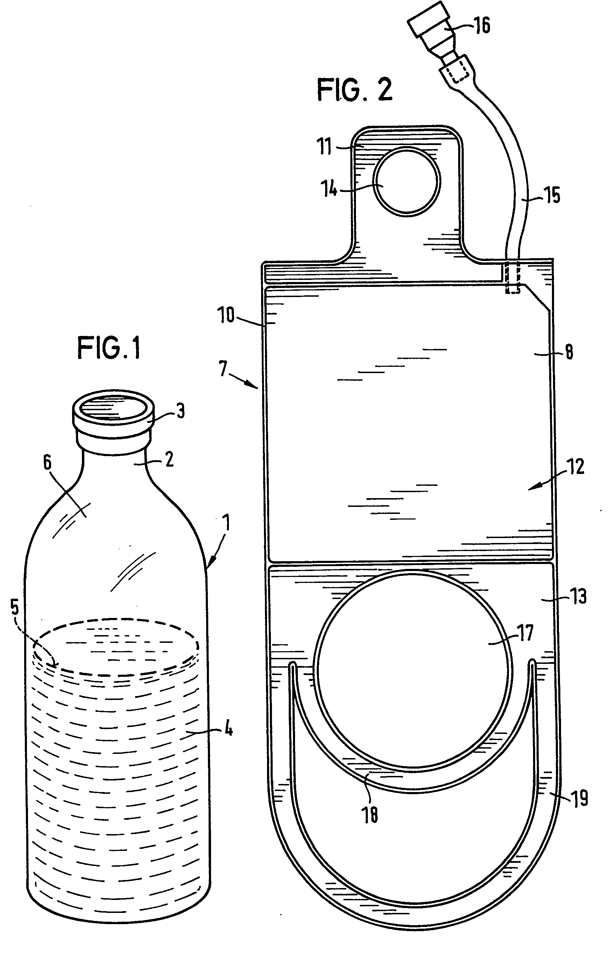

Der in Fig. 1 dargestellte starre Behälter 1 ist eine aus Glas bestehende Infusionsflasche mit einem verengten Hals 2, auf dem ein Verschluß 3 angebracht ist, der die Infusionsflasche luft- und vakuumdicht verschließt, jedoch von einem Anstechdorn eines Infusionsbesteckes, wie weiter unten erläutert, durchstochen werden kann.

Der Behälter 1 ist zu etwas mehr als der Hälfte mit einer Flüssigkeit 4 gefüllt. Bei der in Fig. 1 gezeigten aufrechten Position des Behälters 1 befindet sich oberhalb des Flüssigkeitsspiegels 5 im Behälter 1 ein Hohlraum 6, der nach Einfüllen der Flüssigkeit 4 evakuiert wird und ein Volumen aufweist, das ausreicht, um die in dem flexiblen Kunststoff-Beutel aufbewahrte zweite Flüssigkeit aufzunehmen, damit die beiden Flüssigkeiten miteinander vermischt werden und beispielsweise eine Infusionslösung bilden.

Der in Fig. 2 gezeigte flexible Beutel 7 besteht aus zwei thermoplastischen Kunststoff-Folien 8 und 9, die entlang Schweißnähten 10 dauerhaft miteinander verbunden sind. Durch die Schweißnähte 10 ist der Beutel 7 in drei Hauptabschnitte 11, 12 und 13 unterteilt.

Der Abschnitt 11 ist als zungenförmige Lasche ausgebildet, die eine Öffnung 14 enthält, welche auf den verengten Hals 2 des starren Behälters 1 paßt.

Der mittlere Abschnitt 12 des Beutels 7 dient zur Aufnahme der zweiten Flüssigkeit, die durch einen Schlauch 15 eingefüllt werden kann. Am äußeren Ende des Schlauches 15 ist ein Verschluß 16 angebracht, der ähnlich wie der Verschluß 3 des Behälters 3 von einem Anstechdorn eines Infusionsbesteckes durchstochen werden kann, um die in den mittleren Abschnitt 12 des Beutels 7 eingefüllte Flüssigkeit in den starren Behälter 1 zum Herstellen der gewünschten Infusionslösung zu überführen.

Der dritte Abschnitt 13 ist wiederum als Lasche ausgebildet und enthält eine Öffnung 17, die auf den Hauptabschnitt des Behälters 1 paßt und diesen mit einem ringförmigen Steg 18 umfaßt. Außerdem ist der laschenartige Abschnitt 13 mit einer bogenförmigen Schlaufe 19 versehen, die als Handgriff und als Aufhängeeinrichtung dient.

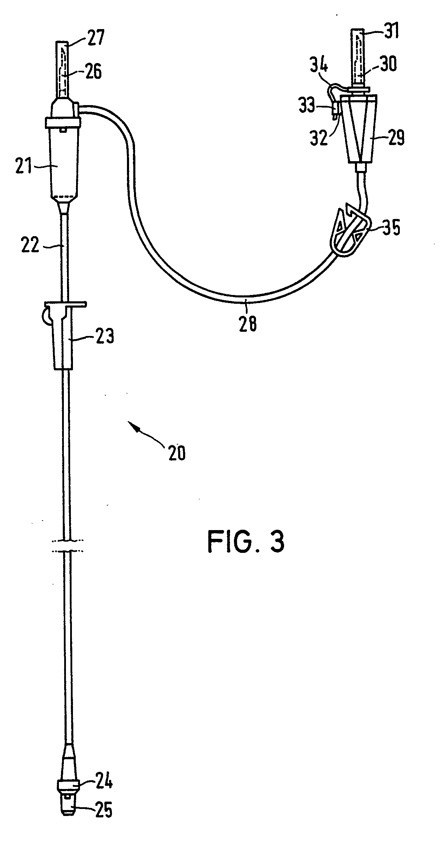

Das in Fig. 3 dargestellte Infusionsbesteck 20 weist eine Tropfkammer 21, einen an diese angeschlossenen Infusionsschlauch 22 mit darauf aufgebrachtem Tropfregler 23 und einen an dem Infusionsschlauch 13 angebrachten Nadeladapter 24 auf, der gemäß Figur 3 mittels einer abnehmbaren Schutzkappe 25 steril abgedeckt ist.

An dem dem Infusionsschlauch 22 gegenüberliegenden Ende ist an die Tropfkammer 21 ein Einstechdorn 26 angeschlossen, der gemäß Fig. 3 von einer abnehmbaren Schutzkappe 27 steril abgedeckt ist.

An die Tropfkammer 21 ist ein weiterer flexibler Schlauch 28 angeschlossen, an dessen äußeren Ende ein verschließbares Lufteinlaßfilter 29 vorgesehen ist. An der Außenseite des Lufteinlaßfilters 29 befindet sich ein weiterer Einstechdorn 30, der gemäß Fig. 3 mittels einer abnehmbaren Schutzkappe 31 steril abgedeckt ist.

Das Lufteinlaßfilter 29 ist mit einer seitlichen Öffnung 32 versehen, die mittels einer Stopfen-Kappe 33 luftdicht zu verschließen ist, welche mittels einer Lasche 34 unverlierbar am unteren Ende des Einstechdornes 30 gehalten ist.

Auf dem Schlauch 28 ist ein üblicher Strömungsregler 35 angebracht, der den Schlauch 28 mehr oder weniger stark zusammenklemmen kann, um dessen lichten Strömungsquerschnitt gegebenenfalls zu reduzieren und damit den Strömungsmitteldurchstrom drosseln zu können.

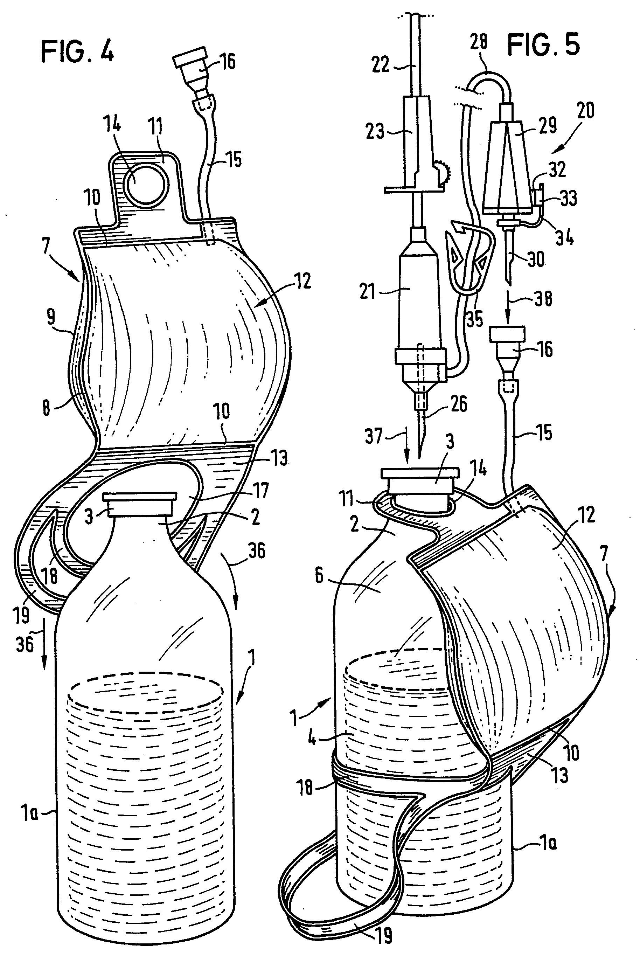

Aus Fig. 4 und 5 ist zu erkennen, wie der flexible Beutel 7 auf den starren Behälter 1 angebracht wird. Zunächst zieht man dem laschenartigen Abschnitt 13 des Beutels 7 in Richtung der Pfeile 36 von oben über den Hals 2 des Behälters 1 derart, daß die Öffnung 17 über den verengten Hals 2 des Behälters 1 hinweg nach unten auf den Hauptteil la des Behälters 1 gelangt und diesen schließlich mit dem Steg 18 ringförmig bzw. kragenförmig umschließt. Dann wird der Abschnitt 11 auf den Hals 2 des Behälters 1 aufgesteckt, so daß der Hals durch die Öffnung 14 hindurchragt und der Abschnitt 11 unterhalb des Verschlusses 3 des Behälters 1 liegt, wie Fig. 5 zeigt.

Aus Fig. 5 ist erkennbar, daß der Beutel 7 rucksackartig oder huckepack auf dem flaschenartigen Behälter 1 angebracht ist. Die Öffnung 17 im laschenartigen Abschnitt 13 des Beutels 7 ist so bemessen, daß sie mit engem Sitz auf den Hauptteil la des Behälters 1 paßt und diesen ringförmig fest umschließt. Der Reibungsschluß zwischen Behälter 1 und dem laschenartigen Abschnitt 13 bzw. dessen Steg 18 ist, selbst wenn der Behälter 1 eine Glasflasche ist, aureichend fest, um ein Verrutschen des Behälters 1 gegenüber dem Beutel zu verhindern. Vielmehr kann man den Behälter 1 mit daran angebrachtem Beutel 7 auch umgekehrt tragen, indem man die Schlaufe 19 als Handgriff benutzt. Hinzu kommt, daß die Öffnung 14 lediglich auf den verengten Hals 2 des Behälters 1 paßt und der Behälter dementsprechend durch diese Öffnung nicht weiter hindurchrutschen kann.

Fig. 5 zeigt, daß Behälter 1 und Beutel 7 eine verhältnismäßig kompakte Einheit bilden, wobei der Beutelabschnitt 12 mit einer Flüssigkeit gefüllt ist, die mit Hilfe des Infusionsbesteckes 20 in der nachstehend beschriebenen Weise in den Behälter 1 übergeleitet werden kann.

Der Behälter 1 mit dem daran angebrachten Beutel 7 wird in der in Fig. 5 dargestellten Position aufgestellt. Dann entfernt man von den Anstechdornen 26 und 30 die Schutzkappen, um die Anstechdorne in den Stopfen-Verschluß 3 des Behälters 1 bzw. den Verschluß 16 des Schlauches 15 gemäß den in Fig. 5 angedeuteten Pfeilen 37 und 38 einzustechen. Dadurch wird eine Verbindung zwischen den Innenräumen des Behälters 1 und des Beutels 7 hergestellt. Wegen des im Hohlraum 6 des Behälters 1 herrschenden Unterdruckes bzw. Vakuums wird die im Beutelabschnitt 12 befindliche Flüssigkeit durch den die Verbindung herstellenden Schlauch 28 in den Behälter 1 eingesaugt, bis der Beutel 7 praktisch leer ist. Dabei erfolgt eine Vermischung zwischen der im Behälter 1 befindlichen Flüssigkeit 4 und der eingesaugten Flüssigkeit.

Nunmehr kann die Vorrichtung zum Verabreichen einer Infusion benutzt werden. Zu diesem Zweck wird sie mit der Schlaufe 19 ergriffen und an einem Haken 39 angehängt, der sich an einem in der Zeichnung nicht dargestellten Ständer befindet oder an anderer Stelle befestigt ist. Dabei wird die Vorrichtung sozusagen auf den Kopf gestellt, wodurch eine weitere Durchmischung der beiden im Behälter 1 befindlichen Flüssigkeiten erfolgt.

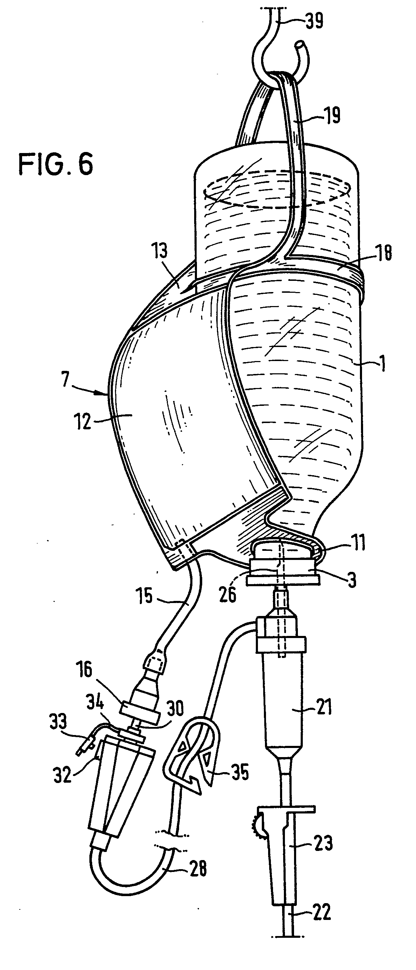

Nun kann die Schutzkappe 25 von dem Nadeladapter 24 abgenommen und eine Infusionsnadel angeschlossen werden, die man am Patienten in üblicher Weise anbringt. Dann braucht nur noch das Lufteinlaßfilter 29 geöffnet werden, wozu man den Deckel 33 von der Öffnung 32 löst, wie in Fig. 6 dargestellt. Dadurch wird der bis dahin im System herrschende Unterdruck aufgehoben, so daß die Infusionsflüssigkeit aus dem Behälter 1 austropfen und durch die Tropfkammer 21 in den Infusionsschlauch 22 gelangen kann.

Mit Hilfe des Tropfreglers 23 läßt sich die Infusionsgeschwindigkeit steuern.

Mit der erfindungsgemäßen Vorrichtung ist es möglich, zwei oder auch mehr Flüssigkeiten unmittelbar vor Verabreichung homogen miteinander zu vermischen und den zum Mischen benutzten Behälter direkt als Infusionsbehälter bzw. Verabreichungsbehälter zu verwenden, ohne die Schlauchverbindung zum flexiblen Beutel lösen und ein separates Infusionsbesteck einführen zu müssen, was Sterilitätsprobleme aufwerfen könnte.

Der flexible Beutel 7 ist etwa rechteckig ausgebildet und besteht vorzugsweise aus zwei an den Kanten miteinander verschweißten flexiblen Kunststoff-Folien, wobei weitere querverlaufende Schweißnähte die einzelnen Abschnitte des Beutels begrenzen. Das Fassungsvermögen des mittleren Abschnittes 12 des Beutels 7 beträgt bei einer Ausführungsform der Erfindung etwa 250 bis 300 ml. Die Laschen und Aufhängeeinrichtungen bestehen aus demselben Material wie der mittlere Abschnitt 12 des Beutels und sind beim dargestellten Ausführungsbeispiel Verlängerungen bzw. Teile der miteinander verschweißten Folien.

Die erfindungsgemäße Vorrichtung kann derart benutzt werden, daß praktisch in einer Folge die beiden Flüssigkeiten miteinander vermischt und dann in Form einer Infusionslösung einem Patienten verabreicht werden. Während des Einstechens der Anstechdorne in die Stopfen-Verschlüsse ist der Infusionsschlauch 22 mittels des Strömungsreglers 23 verschlossen. Dieser Strömungsregler 23 wird erst geöffnet, nachdem das Belüftungsventil 29 geöffnet worden ist und die Verabreichung der Infusionsflüssigkeit beginnen soll.

**Patentansprüche**

1. Vorrichtung mit zwei unmittelbar vor dem Verabreichen steril miteinander zu vermischenden aufbewahrten Flüssigkeiten wie parenteral zu verabreichende Infusionslösungen, mit einem ersten die eine Flüssigkeit aufbewahrenden Behälter (12), der eine Aufhängeeinrichtung (19) und einen Stopfen-Verschluß (16) aufweist, und mit einem zweiten die zweite Flüssigkeit (4) aufbewahrenden Behälter (1), der ebenfalls mit einem Stopfen-Verschluß (3) versehen ist, wobei durch die Stopfen-Verschlüsse (3; 16) beider Behälter (1; 12) je ein Anstechdorn (26; 30) eines Infusionsbesteckes (20) durchstechbar ist, dadurch gekennzeichnet, daß der erste Behälter (12) ein flexibler Beutel (7) aus Kunststoff ist, daß der flexible Behälter (12) mit zwei zum Aufstecken auf den starren Behälter (1) bestimmten Laschen (11, 13), die Öffnungen (14, 17) enthalten, welche den starren Behälter (1) aufnehmen können, und ferner mit einer zum Aufhängen der Vorrichtung geeigneten Schlaufe (19) versehen ist, und daß der zweite Behälter (1) ein separater, vakuumdicht verschließbarer starrer Behälter ist, der ein derartiges Fassungsvermögen bzw. Volumen aufweist, daß er das Gesamtvolumen beider Flüssigkeiten zum Vermischen derselben aufnehmen kann.

2. Vorrichtung nach Anspruch 1, dadurch gekennzeichnet, daß die beiden zur Aufnahme des starren Behälters (1) bestimmten Laschen (11, 13) an entgegengesetzten Enden des rechteckig ausgebildeten flexiblen Behälters (12) vorgesehen sind und sich neben der einen Lasche (11) der Stopfen-Verschluß (16) des flexiblen Behälters (12) befindet.

3. Vorrichtung nach Anspruch 1 oder 2, dadurch gekennzeichnet, daß der starre Behälter (1) flaschenförmig ausgebildet ist und die neben dem Stopfen-Verschluß (16) des flexiblen Behälters (12) befindliche Lasche (11) eine zur Aufnahme des Flaschenhalses (2) des starren Behälters (1) bestimmte Öffnung (14) enthält, die enger als die zur Aufnahme des Bauches (la) des starren Behälters (1) bestimmte Öffnung (17) in der anderen Lasche (13) ist.

4. Vorrichtung nach Anspruch 3, dadurch gekennzeichnet, daß der starre Behälter (1) eine Glasflasche ist.

5. Vorrichtung nach Anspruch 1, dadurch gekennzeichnet, daß die als Aufhängeeinrichtung dienende Schlaufe (19) halbkreisförmig ausgebildet ist.

6. Vorrichtung nach Anspruch 5, dadurch gekennzeichnet, daß sich die halbkreisförmige Schlaufe (19) in der Ebene der Lasche (13) mit der größeren Öffnung (17) befindet und über diese Lasche übersteht.

**Revendications**

1. Dispositif avec deux liquides conservés à mélanger de façon stérile avant l'administration comme des solutions injectables à administrer par voie parentérale, avec un premier récipient (12) conservant un liquide, qui présente un dispositif de suspension (19) et une fermeture-bouchon (16) et avec un deuxième récipient (1) conservant le second liquide (4), qui est également muni d'une fermeture-bouchon (3) où une aiguille (26; 30) d'un ensemble de transfusion (20) peut être piquée à travers les fermetures-bouchons (3; 16) des deux récipients,

caractérisé en ce que le premier récipient (12) est une poche flexible (7) de matière synthétique, en ce que le récipient flexible (12) est muni de deux éclisses (11; 13) prévues pour la fixation sur le récipient rigide (1), et qui présentent des ouvertures (14; 17), qui peuvent se ficher sur le récipient rigide (1) et en outre d'une boucle (19) appropriée pour suspendre le dispositif, et en ce que le second récipient (1) est un récipient rigide séparé, pouvant être fermé de manière à tenir le vide, et qui présente une capacité ou un volume tels qu'il puisse recevoir le volume total des deux liquides afin de les mélanger.

2. Dispositif selon la revendication 1, caractérisé en ce que les deux éclisses (11; 13) prévues pour recevoir le récipient rigide (1) sont prévues à des extrémités opposées du récipient flexible de forme rectangulaire (12), et que près d'une éclisse (11) se trouve la fermeture-bouchon (16) du récipient flexible (12).

3. Dispositif selon la revendication 1 ou 2, caractérisé en ce que le récipient rigide (1) est en forme de flacon, et en ce que l'éclisse (11) qui se trouve près de la fermeture-bouchon (16) du récipient flexible (12) contient une ouverture (14) déterminée pour se prendre autour du col de flacon (2) du récipient rigide (1), qui est plus étroite que l'ouverture (17) prévue pour se prendre autour du ventre (1a) du récipient rigide (1), dans l'autre éclisse (13).

4. Dispositif selon la revendication 3, caractérisé en ce que le récipient rigide (1) est un flacon de verre.

5. Dispositif selon la revendication 1, caractérisé en ce que la boucle servant de dispositif de suspension (19) est de forme semi-circulaire.

6. Dispositif selon la revendication 5, caractérisé en ce que la boucle (19) de forme semi-circulaire se trouve dans le plan de l'éclisse (13) avec la plus grande ouverture (17) et la surmonte.

**Claims**

1. A device with two stored liquids to be mixed together in a sterile manner directly before administration, such as parenteral infusion solutions to be administered, with a first container (12) storing the one liquid and comprising a hanging device (19) and a stopper closure (16), and with a second container (1) for storing the second liquid (4) which is likewise provided with a stopper closure (2), a respective injection needle (26; 30) of an infusion set (20) being insertable through the stopper closures (3; 16) of both containers (1; 12),

characterized in that said first container (12) is a flexible bag (7) made of plastic material, that the flexible bag (12) is provided with two tags (11, 13) designed for fitting onto the rigid container (1), said tags having openings (14, 17) which can receive said rigid container (1), and further with a loop (19) suitable for hanging the device, and that said second container (1) is a separate vacuum-tight closable rigid container having such a capacity and volume, respectively, to receive the total volume of both liquids to be mixed.

2. The device as claimed in claim 1, characterized in that the two tags (11, 13) designed to receive the rigid container (1) are provided on opposite ends of the rectangular flexible container (12), and the stopper closure (16) of the flexible container (12) is near the one tag (11).

3. The device as claimed in claim 1 or 2, characterized in the rigid container (1) is in the form of a flask and the tag (11) which is near the stopper closure (16) of the flexible container (12) contains an opening (14) designed to receive the flask neck (2) of said rigid container (1), this opening being narrower than the opening (17) in the other tag (13) which is designed to receive the belly (1a) of the rigid container (1).

4. The device as claimed in claim 3, characterized in that said rigid container (1) is a glass flask.

5. The device as claimed in claim 1, characterized in that said loop (19) serving as a hanging device is made semi-circular.

6. The device as claimed in claim 5, characterized in that said semi-circular loop (13) is in the plane of the tag (13) having the large opening (17) and extends beyond this tag.

FIG. 2
16
11
14
15
10
8
7
12
13
17
18
19
FIG.1
3
2
6
1
5
4

27
26
21
22
23
20
24
25
31
34
30
33
32
29
35
28

FIG. 3

FIG. 4
16
14
11
15
10
7
12
9
8
10
13
17
3
2
18
36
19
36
1
1a
FIG. 5
22
28
23
20
29
32
33
34
30
21
35
38
16
26
37
15
3
14
11
2
12
6
7
1
4
10
13
18
1a
19

FIG. 6
39
19
13
18
1
7
12
11
3
26
15
16
34
30
33
32
35
21
23
28
22